# EUROPEAN PATENT APPLICATION

(11) **EP 2 727 995 A2**
(43) Date of publication of application: **07.05.2014**
(21) Application number: 12804322.1
(22) Date of filing: 28.06.2012
(51) Int. Cl.: C12N 1/20, C12N 9/02, C12N 15/53, C12P 17/00

(54) **KITASATOSPORA SP. DG09 STRAIN AND METHOD FOR PRODUCING 5,6-DIHYDROXYINDOLE USING SAME**

(30) Priority: 28.06.2011 KR 20110063137
(71) Applicant: Gfc Co., Ltd., Suwon Si, Gyeonggi-Do 443-702 (KR)
(72) Inventor: LEE, Dong Geol, Suwon-si Gyeonggi-do 443-702 (KR); KANG, Hee Cheol, Suwon-si Gyeonggi-do 443-400 (KR); HAN, Sang Keun, Cheonan-si Chungcheongnam-do 330-250 (KR); LEE, Chan Woo, Seongnam-si Gyeonggi-do 463-010 (KR)
(74) Representative: Höglund, Lars
(86) International application number: PCT/KR2012/005099
(87) International publication number: WO 2013/002561

(57) **Abstract**

The present invention relates to a *Kitasatospora sp.* DG09 strain, to a tyrosinase produced from the same, and to a method for producing 5,6-dihydroxyindole using the same. More particularly, the present invention relates to a *Kitasatospora sp.* DG09 strain isolated from soil, to the tyrosinase produced by the strain, and to a method for producing 5,6-dihydroxyindole using the same.

## Description

### Technical Field

The present invention relates to a *Kitasatospora sp.* DG09 strain, tyrosinase produced from the same, and a method for producing 5,6-dihydroxyindole using the same. More particularly, the present invention relates to a *Kitasatospora sp.* DG09 strain isolated from soil, tyrosinase produced from the strain, and a method for producing 5,6-dihydroxyindole using the same.

### Background Art

The hair shaft is a third region of hair that protrudes out of the skin of the scalp, and is made up of the cuticle, the cortex and the medulla. The cortex surrounding the medulla corresponds to the thickest portion (80∼90%) of each hair, and contains granules (particulate) called melanin as a natural pigment. Melanin is synthesized in melanosomes, which are small organelles within melanocytes, and is then transferred to surrounding keratinocytes via melanocyte dendrites. In a pathway of synthesizing melanin, tyrosine is oxidized to DOPA (dihydroxyphenylalanine), which is then oxidized to form Dopaquinone in a process catalyzed by tyrosinase. Subsequently, the reaction proceeds auto-oxidatively, and further accelerated in the presence of an enzyme, as known in the art. Melanin is produced in the human body or hair in order to remove active oxygen or free radicals generated naturally or in order to block ultraviolet light.

The starting material for producing melanin is tyrosine, which is a normal amino acid in the human body.

Tyrosine is oxidized by tyrosinase in melanocytes to yield 3,4-dihydroxyphenylalanine (herein referred to as "DOPA"), which is then further oxidized to form Dopaquinone. After that, Dopaquinone is auto-oxidized to form 5,6-dihydroxyindole, which is then converted into indole-5,6-quinone, thereby finally producing dark brown colored melanin. There are various natural chemical materials comprised of proteins in the human body. Each amino acid has an inherent function. Particularly, tyrosine, which is an amino acid related to colors, and is used to form black melanin, red melanin, mixed melanin, and the like. Melanin forms granules and determines hair color, depending on factors such as type and concentration of melanin. Therefore, every person exhibits a characteristic hair color. Namely, various hair colors such as black, red, blonde, and the like may be determined according to sorts and size of granules of melanin. Eumelanin or tyrosine, which are two sorts of melanin pigment grains residing in the cortex in the middle layer of hair, are the most common and darkest in color, producing brown and black colored hair. Pheomelanin has a lighter shade and produces blonde and red hair. Red hair is caused by a red pigment and a black pigment, and blonde hair is caused by a mixture of the red pigment and the yellow pigment. Sandy brown hair is a mixture of a red pigment, a brown pigment, and a black pigment. Dark brown hair has much more black pigment than sandy brown hair. Grey hair has no pigment grains, and thus appears white.

Melanin complicatedly modifies proteins generated naturally through adjustment of a specific enzyme found in cells producing pigments. It is believed that the different colors of melanin pigments are various stages in a melanin development procedure. Firstly, a yellow pigment is formed, which is then enzymatically converted into a red pigment. The red pigment is converted into a black pigment by means of a different enzyme. Not every person is capable of changing melanin pigments corresponding to general colors. Some people generate only black pigment. Those people come to have black hair including dark brown depending on how much black pigment they produce. Further, since some people only synthesize red or yellow pigments, those people come to have red hair or blonde hair. Capability of generating such pigments is determined by existing pigments, which are exhibited by individual genetic information.

Melanin is a protein which is insoluble in water and is a determinant of hair color. Tyrosine which is an amino acid produced by hair papillae is an oval-shaped precursor to melanin and has a major diameter of 0.8∼1.8 µm and a minor diameter of 0.3∼0.4 µm When melanin production ceases at the level of Dopaquinone, hair is keratinized, and hair turns grey. Namely, grey hair refers to a state wherein oxidation of tyrosine is not performed since the enzyme tyrosinase is not expressed. Grey hair is particularly prevalent among persons with dark brown hair. Further, grey hair is a sort of aging phenomenon stemmed from cease of melanin production in melanocytes.

Dopaquinone undergoes reaction via two pathways. One pathway is that dopaquinone is converted into dopachrome, which then leads to the production of eumelanin having a dark brown color. The other pathway is that dopaquinone binds to cysteine present in keratin proteins, which then leads to the production of pheomelanin having a red brown color. Such an oxidative pigmentation reaction of hair is an example of polymerization (binding) in hair. In this reaction, an aromatic amine and a phenol monomer undergo oxidative polymerization, thereby binding with an amino acid in hair. Eumelanin has a relatively large in size and is highly liable to be chemically degraded, whereas pheomelanin has a relatively small in size and exhibits strong chemical stability. As hair contains large amounts of cysteine, large amounts of pheomelanin are present in hair. The red shade of hair when dark brown color hair is bleached with H₂O₂ is due to the fact that eumelanin is oxidatively degraded while pheomelanin remains unchanged.

The present inventors produced a raw material for hair dyes in order to improve upon demerits of existing hair dyes, such as hair dye induced skin irritation and difficulty in effective dying using the hair dyes, by utilizing a recombinant strain using 5,6-dihydroxyindole which is a precursor to melanin. As a result of evaluation with respect to volunteer hair samples, it was found that the raw material has considerable efficacy in hair dying. The present inventors completed the present invention by establishing *Escherichia coli* transformed with a recombinant plasmid vector of the present invention and a method for producing 5,6-dihydroxyindole using the same.

### Brief description of the Drawings

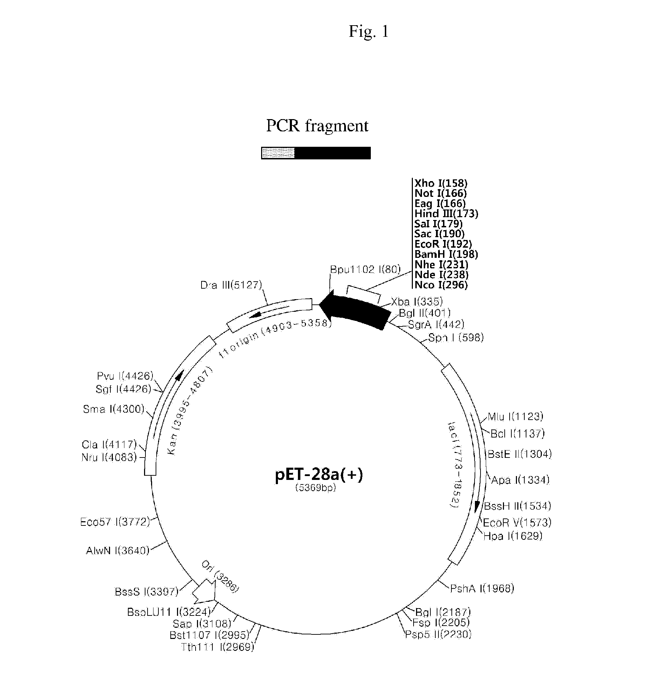

Fig. 1 shows a recombinant vector including a gene encoding the tyrosinase in accordance with an embodiment of the present invention.

### Disclosure of the invention

### Technical Problem

It is an object of the present invention to provide a *Kitasatospora sp.* DG09 strain and a novel tyrosinase produced from the strain.

It is another object of the present invention to provide a method for producing 5,6-dihydroxyindole using the strain and the tyrosinase produced from the strain.

### Technical Solution

The present invention provides a *Kitasatospora sp.* DG09 JF917133 strain, which is deposited at the KCCM (Korea Culture Center of Microorganisms) under deposition No. KCCM11178P, and produces a tyrosinase.

The present invention provides a tyrosinase produced from the *Kitasatospora sp.* DG09 strain.

In one embodiment of the present invention, the tyrosinase may have the amino acid sequence of SEQ ID NO: 2.

In one embodiment of the present invention, the tyrosinase may have the nucleic acid sequence of SEQ ID NO: 3.

The present invention provides a gene encoding the tyrosinase.

The present invention provides a recombinant vector comprising the gene, and a transformant into which the recombinant vector is introduced.

The present invention provides a method for preparing 5,6-dihydroxyindole, which includes: culturing the *Kitasatospora sp.* DG09 strain or the transformant; and collecting 5,6-dihydroxyindole from the obtained culture solution.

### Advantageous Effects

The present invention provided a novel *Kitasatospora sp.* strain producing a tyrosinase. Further, the present invention provides a method for producing 5,6-dihydroxyindole using the strain and the tyrosinase produced from the strain.

### Best Mode

The present invention provides a *Kitasatospora sp.* DG09 JF917133 strain producing tyrosinase.

The present inventors isolated a strain having a high yield of tyrosinase production due to high capability of producing melanin from tyrosine, and examined the strain's physiological properties, thereby identifying the strain of the present invention.

In order to identify strains satisfying the aforementioned conditions, the present inventors cultured a dilute solution obtained from soil in LDG media containing tyrosine (Glucose 1g/L, NaCl 5g/L, 0.1 CaCl₂, 10g Tryptone 10g/L, Casein 5g/L, L-tyrosinase 2g/L) and then selected a strain having the highest melanin production capabilities. Then, the base sequence (JF917133) of 16S rRNA of the selected strain was determined. As a result of searching the GenBank database, it was confirmed that the strain had 99% identity with *Kitasatospora sp.* strain. Based on this result, the strain was identified as *Kitasatospora sp.,* designated as *Kitasatospora sp.* DG09' and was assigned accession number JF917133 by GenBank. Further, the selected strain was deposited at an International microorganism depository, the KCCM, and was assigned deposition number KCCM11178P on March 2, 2011.

The *Kitasatospora sp.* DG09 strain may be classified as *Bacteria* kingdom, *Actinobacteria* phylum, *Actinobacteria* class, *Actinomycetales* order, *Streptomycetaceae* family, *Kitasatospora* genus, *Kitasatospora paranensis* species.

The *Kitasatospora sp.* DG09 strain shows culturing properties as listed in Table 1.

**TABLE 1**

| | **ISP2 media** | **ISP3 media** | **ISP4 media** | **ISP5 media** |
|---|---|---|---|---|
| **Degree of Growth** | moderate | abundant | moderate | moderate |
| **Aerial mycelium** | white | gray | gray | gray |
| **Reverse color when the stain arrived at final growth stage** | yellowish brown | brown | brown | brown |

Although the *Kitasatospora sp.* DG09 strain does not produce a soluble pigment, the strain can produce a melanoid pigment in peptone/yeast extract/iron and tyrosine agar.

The *Kitasatospora sp.* DG09 strain produces essentially no submerged spores in liquid culture solution.

The *Kitasatospora sp.* DG09 strain has a growth temperature range from 10°C to 37°C, and an optimum growth temperature range from 25°C to 28°C. The *Kitasatospora sp.* DG09 strain does not grow at 6°C or 40°C.

The *Kitasatospora sp.* DG09 strain has a pH ranging from pH 5.0 to pH 9.0 for good growth. At pH 4.0 or pH 9.5, the strain does not grow.

The *Kitasatospora sp.* DG09 strain is an aerobic, Gram positive, and non-acid-fast actinomycete, which does not produce acidic materials in culture.

The *Kitasatospora sp.* DG09 strain produces yellowish brown to dark brown mycelium and a grey aerial spore mass in glycerol asparagine, inorganic salt/starch, oatmeal and yeast extract/malt extract agar, and does not produce an acidic material.

As can be seen from Example below, we identified that the strain of the present invention has a high yield of tyrosinase production, and the strain can grow well in LDG media (Glucose 1g/L, NaCl 5g/L, CaCl₂ 0.1 g/L, Tryptone 10g/L, Casein 5g/L, L-tyrosinase 2g/L).

As an example of culturing the strain of the present invention, the strain can grow well in an LDG media (Glucose 1g/L, NaCl 5g/L, CaCl₂ 0.1g/L, Tryptone 10g/L, Casein 5g/L, L-tyrosinase 2g/L) at 25°C.

The present invention provides a tyrosinase produced from the *Kitasatospora sp.* DG09 JF917133 strain, and a gene encoding the same.

The present inventors utilized a shotgun cloning technique in order to produce tyrosinase from the *Kitasatospora sp. DG09* JF917133 strain, and performed cloning of the produced tyrosinase. Further, the present inventors amplified the base sequence identified through shotgun cloning via PCR (Polymerase Chain Reaction), and determined the base sequence of the tyrosinase.

The tyrosinase has a main amino acid sequence of SEQ ID NO: 2. The tyrosinase has the nucleic acid sequence of SEQ ID NO: 3. The tyrosinase of the present invention was found to be a new tyrosinase after thoroughly searching the GenBank database. This tyrosinase includes a base sequence which is different from that of the existing tyrosinase (NC_003279.6). Since the base sequence is obtained from a host having a base sequence different from the existing base sequence, the tyrosinase has a different activity. Main differences are a period of time for producing melanin is different, and a color of the finally produced melanin.

The tyrosinase is active at a temperature of 20°C to 35°C, and exhibits maximum activity at 25°C.

The present invention provides a recombinant vector including the gene encoding the tyrosinase, and a transformant into which the recombinant vector is introduced.

The *Kitasatospora sp.* DG09 strain and tyrosinase of the present invention are regarded as novel in view of the base sequence and biochemical properties. Further, the base sequence encoding the tyrosinase isolated from the strain, a recombinant vector including the gene and a transformant into which the recombinant vector is introduced may be manufactured using conventional methods known in the art.

The present invention provides a method for producing 5,6-dihydroxyindole using the *Kitasatospora sp.* DG09 JF917133 strain or the transformant. Specifically, the method for producing 5,6-dihydroxyindole includes: culturing the *Kitasatospora sp.* DG09 strain or the transformant (Step 1); and collecting 5,6-dihydroxyindole from the obtained culture solution (Step 2).

In Step 1, the strain and the transformant may be prepared by the aforementioned method. The media is not particularly limited so long as the media includes tyrosine, but LDG media (Glucose 1g/L, NaCl 5g/L, CaCl₂ 0.1 g/L, Tryptone 10g/L, Casein 5g/L, L-tyrosinase 2g/L) may be utilized. Conditions for culturing the strain or the transformant are not particularly limited, but it is possible to culture the strain or the transformant at about 25°C to 37°C for about two days.

In Step 2, 5,6-dihydroxyindole may be collected from the obtained culture solution. 5,6-dihydroxyindole may be collected through centrifugation.

Next, the present invention will be described in more detail with reference to the following example and drawings. However, it should be understood that the following example is not to be in any way construed as limiting the present invention.

### Example 1

### Isolation and Selection of strain

Soil was taken and stirred in sterilized and distilled water, from which the supernatant solution of the sampled solution was taken and diluted. The obtained diluted solution was blotted onto LDG media containing tyrosine (Glucose 1g/L, NaCl 5g/L, 0.1 CaCl₂, 10g Tryptone 10g/L, Casein 5g/L, L-tyrosinase 2g/L), followed by culturing at 25°C to 37°C for two days, thereby isolating a *Kitasatospora sp.* DG09 JF917133 capable of forming black clusters by oxidizing tyrosine and comprised of a single colony. The isolated microorganism was introduced into LDG media (Glucose 1g/L, NaCl 5g/L, CaCl₂ 0.1 g/L, Tryptone 10g/L, Casein 5g/L, L-DOPA 3g/L) containing added L-DOPA, and cultured at 25°C for 48 hours. After culturing, a strain showing a maximum melanin production rate was selected by measuring absorbance at 530 nm as the degree of forming a black cluster, namely melanin.

### Identification of the Isolated Strain

In order to identify a final strain isolated from the soil, the base sequence of 16S rDNA was examined. In order to determine the base sequence of 16S rDNA of the isolated strain, genomic DNA of the strain was isolated and amplified by PCR. PCR was performed in a 50 µl solution with the following components. To 1 µl of genomic DNA (50 ∼ 100 ng/µl), 2 µl of 10 X Taq DNA polymerase buffered solution (with added MgCl₂), 2 µl of 2.5 mM dNTPs, 1 µl of each of 10 pmol of forward primer (27f: 5'-AGAGTTTGATCCTGGCTCAG-3': SEQ ID NO: 4) and reverse primer (1492r: 5'-TACGGYTACCTTGTTACGACTT-3', SEQ ID NO: 5), and 1-2 units of Tag DNA polymerase (Promega, USA) were added together with distilled water to a final reaction solution volume of 50 µl. The primers were synthesized for use in amplification of a polynucleotide corresponding to the 16s rDNA portion of a prokaryotic cell. PCR reaction consisted of 30 cycles of denaturation at 95°C for 5 minutes, denaturation at 95°C for 30 seconds, annealing at 52°C for 45 seconds, and elongation at 72°C for 45 seconds, and final elongation at 72°C for 8 minutes and final hold at 4°C. As a result, the base sequence of the amplified fragment was determined, thereby obtaining a base sequence of SEQ ID NO: 1 (JF917133). By searching the GenBank database, the base sequence showed 98% identity with *Kitasatospora sp.* strain. Accordingly, the isolated strain was identified as belonging to *Kitasatospora sp.* and designated as *'Kitasatospora sp.* DG09'. The *Kitasatospora sp.* DG09 strain was deposited with the KCCM and assigned deposition No. KCCM 11178P on March 2, 2011.

### Isolation and Purification of tyrosinase

The tyrosinase produced from the strain was isolated as follows, and then the amino acid and base sequence were determined.

Specifically, the strain was analyzed as follows.

The amino acid sequence of the isolated tyrosinase was analyzed using the NCBI BLAST software package. It was confirmed that the identified tyrosinase had the amino acid sequence of JF917133.

The base sequence of the isolated tyrosinase was analyzed using the NCBI BLAST software package. It was confirmed that the tyrosinase identified thusly had the base sequence of JF917133.

### Preparation of Recombinant Vector and Transformant

Nucleic acid extracted from the *Kitasatospora sp.* DG09 strain was introduced to *Escherichia coli* to perform the recombination. The recombinant vector was not particularly limited, but pET-28a vector was employed.

Specifically, both the pUC19 vector (Invitrogen USA) used during recombination and the nucleic acid extracted from the isolated *Kitasatospora sp.* DG09 were treated with restriction enzymes ECOR1 and BamH1 at 37°C for two hours. The size of the nucleic acid (about 10 Kb) and pUC 19 vector (about 2.6 Kb) restriction enzyme fragments were identified through electrophoresis, and then purified using a nucleic acid purification kit. The purified nucleic acid was subjected to recombination using T4 ligase at 19°C for 12 hours. The size of the nucleic acid and vector upon which recombination was completed was identified through electrophoresis. It was identified that the size was about 12 Kb.

The recombinant pUC 19 vector identified by electrophoresis was introduced into DH5a *Escherichia coli* to perform transformation through heat shock. The transformed *Escherichia coli* was cultured in an LB medium for about 12 hours. After culturing for 12 hours, colonies having white clusters were separately selected, followed by culturing in LB medium. The cultured solution was subjected to centrifugation to collect microbial cells. From the microbial cells, a plasmid was isolated. The isolated plasmid was cut with restriction enzymes EcoR1 and BamH1, and the cut nucleic acid was subjected to electrophoresis to determine the size thereof. A nucleic acid having about 2 kb was separately isolated and purified. The purified nucleic acid was introduced into PET28a vector to perform recombination again. One example of the prepared recombinant vector is illustrated in Fig. 1. The recombinant PET28a vector was inserted into BL21 (DE3) *Escherichia coli* to perform transformation, followed by culturing at 28°C in an LB medium for about 12 hours. 1 mM of IPTG was used to induce protein expression of the gene.

### Production of 5,6-dihydroxyindole

5,6-dihydroxyindole was produced by reacting the culture solution produced from *Kitasatospora sp.* DG09 strain with L-DOPA for about 1 hour. The reaction with oxygen was carried out in a sealed container in order to prevent natural oxidation through reaction with carbon dioxide and oxygen. After the reaction, the reaction solution was concentrated in order to remove water and other byproducts. The concentrated product was calibrated by using an amine type solution as an antioxidant, and NaOH to raise pH.

### Free text for Sequence Listing

SEQ ID NO: 1: 16S rDNA sequence *of Kitasatospora sp.* DG09 strain
SEQ ID NO: 2: Amino acid sequence *of tyrosinase*
SEQ ID NO: 3: Nucleic acid sequence *of tyrosinase*
SEQ ID NO: 4: Forward primer sequence
SEQ ID NO: 5: Reverse primer sequence

<110>     International GFC

<120>     Kitasatospora sp. and a method for preparing 5,6-dihydroxyindole
          using the same

<130>     AJP11086

<160>     5

<170>     KopatentIn 2.0

<210>     1
<211>     1377
<212>     DNA
<213>     16S rDNA sequence of Kitasatospora sp. DG09

<400>     1
tccctccaag ggttaggcca ccggcttcgg gtgttaccga ctttcgtgac gtgacgggcg        60

gtgtgtacaa ggcccgggaa cgtattcacc gcagcatgct gatctgcgat tactagcaac       120

tccaacttca tggggtcgag ttgcagaccc caatccgaac tgagaccggc tttttgggat       180

tcgctccgcc tcgcggcatc gcagcccttt gtaccggcca ttgtagcacg tgtgcagccc       240

aagacataag gggcatgatg atttgacgtc gtccccacct tcctccgagt tgaccccggc       300

agtctcctgt gagtccccga cattactcgc tggcaacaca gaacaagggt tgcgctcgtt       360

gcgggactta acccaacatc tcacgacacg agctgacgac aaccatgcac cacctgtaca       420

ccgaccacaa gggggcgacc atctctggcc gtttccggtg tatgtcaagc cttggtaagg       480

ttcttcgcgt tgcgtcgaat taagccacat gctccgctgc ttgtgcgggc ccccgtcaat       540

tcctttgagt tttagccttg cggccgtact ccccaggcgg ggaacttaat gcgttagctg       600

cggcaccgac gacgtggaat gtcgccaaca cctagttccc aacgtttacg gcgtggacta       660

ccagggtatc taatcctgtt cgctccccac gctttcgctc ctcagcgtca gtaatggccc       720

agagatccgc cttcgccacc ggtgttcctc ctgatatctg cgcatttcac cgctacacca       780

ggaattccga tctcccctac cacactccag cctgcccgta tcgaatgcag acccggggtt       840

aagccccggg ctttcacatc cgacgcgaca ggccgcctac gagctcttta cgcccaataa       900

ttccnggaca acgctcgcac cctacgtatt accgcggctg ctggcacgta gttagccggt       960

gcttcttctg caggtaccgt cacttgcgct tcttccctgc tgaaagaggt ttacaacccg      1020

aaggccgtca tccctcacgc ggcgtcgctg catcaggctt tcgcccattg tgcaatattc      1080

cccactgctg cctcccgtag gagtctgggc cgtgtctcag tcccagtgtg gccggtcgcc      1140

ctctcaggcc ggctacccgt cgtcgccttg gtaggccatt accccaccaa caagctgata      1200

ggccgcggga tcatcctgca ccgccggagc tttacaccca cccccatgcg gaggaaggtc      1260

atatccggta ttagacctcg tttccaaggc ttgtcccaga gtgcagggca gatttcccac      1320

gtgttactca cccgttcgcc actgatccac cccgaagggc ttcaccgttc gactgca         1377

<210>    2
<211>    241
<212>    PRT
<213>    amino acid sequence of tyrosinase

<400>    2
Met Leu Ile Cys Asp Tyr Gln Leu Gln Leu His Gly Val Glu Leu Gln
1               5                   10                  15

Thr Pro Ile Arg Thr Glu Thr Gly Phe Leu Gly Phe Ala Pro Pro Arg
            20                  25                  30

Gly Ile Ala Ala Leu Cys Thr Gly His Cys Ser Thr Cys Ala Ala Gln
        35                  40                  45

Asp Ile Arg Gly Met Met Ile Arg Arg Pro His Leu Pro Pro Ser Pro
    50                  55                  60

Arg Gln Ser Pro Val Ser Pro Arg His Tyr Ser Leu Ala Thr Gln Asn
65                  70                  75                  80

Lys Gly Cys Ala Arg Cys Gly Thr Pro Asn Ile Ser Arg His Glu Leu
                85                  90                  95

Thr Thr Thr Met His His Leu Tyr Thr Asp His Lys Gly Ala Thr Ile
            100                 105                 110

Ser Gly Arg Phe Arg Cys Met Ser Ser Leu Gly Lys Val Leu Arg Val
        115                 120                 125

Ala Ser Asn Ala Thr Cys Ser Ala Ala Cys Ala Gly Pro Arg Gln Phe
    130                 135                 140

Leu Val Leu Ala Leu Arg Pro Tyr Ser Pro Gly Gly Glu Leu Asn Ala
145                 150                 155                 160

Leu Ala Ala Ala Pro Thr Thr Trp Asn Val Ala Asn Thr Phe Pro Thr
                165                 170                 175

Phe Thr Ala Trp Thr Thr Arg Val Ser Asn Pro Val Arg Ser Pro Arg
            180                 185                 190

Phe Arg Ser Ser Ala Ser Val Met Ala Gln Arg Ser Ala Phe Ala Thr
        195                 200                 205

Gly Val Pro Pro Asp Ile Cys Ala Phe His Arg Tyr Thr Arg Asn Ser
    210                 215                 220

Asp Leu Pro Tyr His Thr Pro Ala Cys Pro Tyr Arg Met Gln Thr Arg
225                 230                 235                 240

Gly

<210>    3
<211>    747
<212>    DNA
<213>    nucleic acid sequence of tyrosinase

<400>    3
atgctgatct gcgattacta gcaactccaa cttcatgggg tcgagttgca gaccccaatc        60

cgaactgaga ccggcttttt gggattcgct ccgcctcgcg gcatcgcagc cctttgtacc 120

ggccattgta gcacgtgtgc agcccaagac ataaggggca tgatgatttg acgtcgtccc 180

caccttcctc cgagttgacc ccggcagtct cctgtgagtc cccgacatta ctcgctggca 240

acacagaaca agggttgcgc tcgttgcggg acttaaccca acatctcacg acacgagctg 300

acgacaacca tgcaccacct gtacaccgac cacaaggggg cgaccatctc tggccgtttc 360

cggtgtatgt caagccttgg taaggttctt cgcgttgcgt cgaattaagc cacatgctcc 420

gctgcttgtg cgggcccccg tcaattcctt tgagttttag ccttgcggcc gtactcccca 480

ggcggggaac ttaatgcgtt agctgcggca ccgacgacgt ggaatgtcgc caacacctag 540

ttcccaacgt ttacggcgtg gactaccagg gtatctaatc ctgttcgctc cccacgcttt 600

cgctcctcag cgtcagtaat ggcccagaga tccgccttcg ccaccggtgt tcctcctgat 660

atctgcgcat ttcaccgcta caccaggaat tccgatctcc cctaccacac tccagcctgc 720

ccgtatcgaa tgcagacccg gggttaa 747

<210> 4
<211> 20
<212> DNA
<213> Artificial Sequence

<220>
<223> forward primer of 27f

<400> 4
agagtttgat cctggctcag 20

<210> 5
<211> 22
<212> DNA
<213> Artificial Sequence

<220>
<223> reverse primer of 1492r

<400> 5
tacggytacc ttgttacgac tt 22

## Claims

1. A *Kitasatospora sp.* DG09 strain which is deposited at KCCM (Korea Culture Center of Microorganisms) under deposition No. KCCM11178P, and produces tyrosinase.

2. A tyrosinase produced from the strain according to claim 1.

3. The tyrosinase according to claim 2, wherein the tyrosinase has the amino acid sequence of SEQ ID NO: 2.

4. The tyrosinase according to claim 2, wherein the tyrosinase has the nucleic acid sequence of SEQ ID NO: 3.

5. A method for preparing 5,6-dihydroxyindole, comprising: culturing the *Kitasatospora sp.* DG09 strain according to claim 1 or the transformant comprising a base sequence encoding the tyrosinase according to claim 2; and collecting 5,6-dihydroxyindole from the obtained culture solution.
